# EUROPEAN PATENT APPLICATION

(11) **EP 4 360 752 A1**
(43) Date of publication of application: **01.05.2024**
(21) Application number: 22849754.1
(22) Date of filing: 12.07.2022
(51) Int. Cl.: B01J 21/02, B01J 21/04, B01J 21/08, B01J 23/42, B01J 23/44, B01J 23/46, B01J 23/75, B01J 23/755, B01J 23/72

(54) **CATALYST FOR PREPARING 1,3-CYCLOPENTANEDIOL, METHOD FOR PREPARING 1,3-CYCLOPENTANEDIOL BY USING SAME, AND 1,3-CYCLOPENTANEDIOL PREPARED THEREBY**

(30) Priority: 29.07.2021 KR 20210100038
(71) Applicant: Kolon Industries, Inc., Seoul 07793 (KR)
(72) Inventor: SHIN, Mi, Seoul 07793 (KR); HA, Ji Min, Seoul 07793 (KR); JEONG, Mee Hye, Seoul 07793 (KR); KIM, Jun Yeong, Seoul 07793 (KR); YONG, Da Kyoung, Seoul 07793 (KR); PARK, Ki Hyun, Seoul 07793 (KR)
(74) Representative: Ter Meer Steinmeister & Partner
(86) International application number: PCT/KR2022/010093
(87) International publication number: WO 2023/008778

(57) **Abstract**

Provided is a catalyst for preparing 1,3-cyclopentanediol by hydrogenation of 4-hydroxy-2-cyclopentenone, the catalyst including: a carrier including α-Al₂O₃; and an active metal supported on the carrier.

## Description

### [Technical Field]

The present invention relates to a catalyst for preparing 1,3-cyclopentanediol, a method for preparing 1,3-cyclopentanediol using the same, and 1,3-cyclopentanediol prepared thereby.

### [Background Art]

In general, polymers such as polycarbonate, polyester, and the like are prepared by using raw materials derived from petroleum resources. However, since there has recently been a concern about depletion of the petroleum resources, there is a demand for providing polymer resins by using raw materials obtained from biomass resources such as plants, etc.

In addition, since there have been concerns about climate change by global warming due to increased emission and accumulation of carbon dioxide, there is a need for developing polymers by using plant-derived monomers emitting extremely low carbon during the manufacturing process.

Furfural is an organic compound extracted from plant materials such as corn cobs, oats, bran, rice, bar gas, and sawdust. In other words, the furfural is a biomass-based chemical material, from which 1,3-cyclopentanediol may be prepared.

The 1,3-cyclopentanediol from biomass-derived furfural can be produced in three steps according to Reaction Scheme 1.

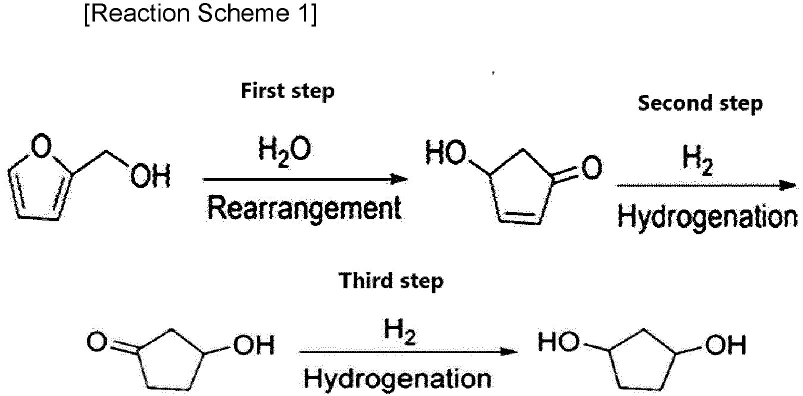

In Reaction Scheme 1, 3-hydroxycyclopentanone, an intermediate is formed by hydrogenation reaction of 4-hydroxy-2-cyclopentenone, the reactant in the second step, and 1,3-cyclopentanediol is formed by hydrogenation reaction of 3-hydroxycyclopentanone.

However, the conventional method for preparing 1,3-cyclopentanediol has problems in that the types of catalysts used in the second and third steps are different, the reaction conditions are complicated, and the reaction time takes a long time. In addition, there is a problem that the yield of 1,3-cyclopentanediol is very low due to the production of intermediates.

Therefore, there is a need for a method for producing 1,3-cyclopentanediol that can solve these problems.

### [Prior Art Documents]

### [Patent Document]

Korean Patent Laid-Open Publication No. 10-2015-0067788

### [Disclosure]

### [Description of the Drawings]

### [Technical Problem]

The purpose of the present invention is to provide a catalyst for preparing 1,3-cyclopentanediol which has fewer acid points on the surface to generate less by-products during the hydrogenation reaction, excellent selectivity for 1,3-cyclopentanediol, and excellent formability to be easily used in the process.

### [Technical Solution]

According to an embodiment, as a catalyst for preparing 1 ,3-cyclopentanediol by hydrogenation of 4-hydroxy-2-cyclopentenone, a catalyst for preparing 1,3-cyclopentanediol is provided, which includes a carrier including α-Al₂O₃ and an active metal supported on the carrier.

The α-Al₂O₃ may have an acid point of 10 mmol/g or less when measured using an ammonia desorption method.

The α-Al₂O₃ may have a specific surface area (BET) of 0.1 m²/g to 50.0 m²/g, a pore volume of 0.10 ml/g to 0.85 ml/g, and a lateral crushing strength of 20 N/particle to 200 N/particle.

The carrier may include α-Al₂O₃, crystalline silica alumina, amorphous silica, amorphous silica alumina, or a combination thereof.

The carrier may include 65 wt% to 99.9 wt% of α-Al₂O₃, and 0.1 wt% to 35 wt% of crystalline silica alumina, amorphous silica, amorphous silica alumina, or a combination thereof, based on a total weight of the carrier.

The active metal may include Pt, Pd, Ru, Ir, Ni, Co, Cu, or a combination thereof.

The catalyst may include 0.1 wt% to 10 wt% of the active metal based on a total weight of the catalyst.

According to another embodiment, a method includes preparing 1,3-cyclopentanediol by hydrogenating 4-hydroxy-2-cyclopentenone in the presence of a catalyst, wherein the catalyst includes a carrier including α-Al₂O₃, and an active metal supported on a carrier.

The preparing of the 1,3-cyclopentanediol may be performed by adding a catalyst to a solution of 4-hydroxy-2-cyclopentenone in an organic solvent.

The 4-hydroxy-2-cyclopentenone may be included in an amount of 0.01 wt% to 50 wt% based on a total weight of the solution.

The organic solvent may include 2-methyltetrahydrofuran, methanol, acetone, or a combination thereof.

In the preparing of 1,3-cyclopentanediol, 0.05 to 0.3 parts by weight of catalyst may be added based on 100 parts by weight of 4-hydroxy-2-cyclopentenone.

The preparing of 1,3-cyclopentanediol may be performed under a hydrogen atmosphere, a temperature of 70 °C to 200 °C, and a pressure of 10 bar to 70 bar.

The preparing of 1,3-cyclopentanediol may be performed for 10 minutes to 2 hours.

The method for preparing 1,3-cyclopentanediol may further include preparing 4-hydroxy-2-cyclopentenone by adding a catalyst to an aqueous solution of furfuryl alcohol before the preparing of 1,3-cyclopentanediol.

The furfuryl alcohol may be a biomass derived compound.

The catalyst may include calcium oxide, lead oxide, aluminum oxide, iron oxide, calcium chloride, zinc acetate, paratoluene sulfonic acid, stannous chloride, stannous sulfate, stannous oxide, stannic oxide, stannous octylate, tetraphenyl tin, a tin powder, titanium tetrachloride, or a combination thereof.

In the preparing of 4-hydroxy-2-cyclopentenone, 0.1 to 10 parts by weight of catalyst may be added based on 100 parts by weight of furfuryl alcohol.

According to another embodiment, 1,3-cyclopentanediol prepared by the above method is provided.
The 1,3-cyclopentanediol may include 1 wt% or less of by-products including cyclopentanone, cyclopentanol, 1,3-cyclopentanediol, or a combination thereof.

### [Advantageous Effects]

The catalyst for preparing 1,3-cyclopentanediol of the present invention has few acid points on the surface, and thus it generates less by-products during hydrogenation reaction, has excellent selectivity for 1,3-cyclopentanediol, and has excellent moldability, making it easy to use in the process.

In addition, the catalyst for preparing 1,3-cyclopentanediol of the present invention does not form the intermediate 3-hydroxycyclopentanone, thereby reducing production steps, and simplifying the production process and reducing reaction time.

### [Mode for Invention]

The advantages and features of the present disclosure and the methods for accomplishing the same will be apparent from the embodiments described hereinafter. However, the embodiments should not be construed as being limited to the embodiments set forth herein. Unless otherwise defined, all terms (including technical and scientific terms) used herein have the same meaning as commonly understood by one of ordinary skill in the art. The terms defined in a generally-used dictionary may not be interpreted ideally or exaggeratedly unless clearly defined.

In addition, unless explicitly described to the contrary, the word "comprise", and variations such as "comprises" or "comprising", will be understood to imply the inclusion of stated elements but not the exclusion of any other elements. Further, the singular includes the plural unless mentioned otherwise.

A catalyst for preparing 1,3-cyclopentanediol according to an embodiment includes a carrier including α-Al₂O₃, and an active metal supported on the carrier.

The catalyst for preparing 1,3-cyclopentanediol produces 1,3-cyclopentanediol through a hydrogenation reaction of 4-hydroxy-2-cyclopentenone.

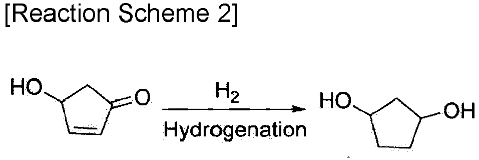

That is, the catalyst for preparing 1,3-cyclopentanediol includes a carrier including α-Al₂O₃ and an active metal supported on the carrier, thereby preparing 1,3-cyclopentanediol directly from 4-hydroxy-2-cyclopentenone without forming the intermediate 3-hydroxycyclopentanone.

In addition, the catalyst for preparing 1,3-cyclopentanediol includes α-Al₂O₃ with few acid points on the surface as a carrier, and thus there are few by-products during the hydrogenation reaction and excellent selectivity for 1,3-cyclopentanediol.

As an example, α-Al₂O₃ may have an acid point of 10 mmol/g or less, for example, 0.1 mmol/g or less, or 0.001 to 10 mmol/g, when measured by ammonia desorption method (NH₃-TPD).

The ammonia desorption method (NH₃-TPD) is a method to measure an amount of acid sites by isothermally adsorbing basic ammonia at 100 °C and then measuring the desorbed ammonia molecules while increasing the temperature at a constant temperature increase rate (e.g., 10 °C/min).

In addition, when α-Al₂O₃ is included as a carrier, the catalyst for preparing 1,3-cyclopentanediol has excellent formability and is easy to use in the process. On the other hand, when using a carbon (C) carrier, catalytic activity is good, but it is difficult to mold in a process, and thus it is mainly used in powder form. The α-Al₂O₃ may be formed into shapes common in the field, such as rings, spheres, columns, porous columns, etc.

The α-Al₂O₃ may have a specific surface area (BET) of 0.1 m²/g to 50.0 m²/g. The specific surface area of α-Al₂O₃ can be measured by the BET method using nitrogen physisorption according to the international test standard ISO-9277. For example, a nitrogen physisorption meter (Quantachrome, NOVA2000e) can be used to measure the specific surface area of α-Al₂O₃. If the specific surface area (BET) of α-Al₂O₃ is less than 0.1 m²/g, it may not be easy to support the metal, and if it exceeds 50.0 m²/g, moisture in the air is easily absorbed, which may reduce the activity of the supported metal.

The α-Al₂O₃ may have a pore volume of 0.10 ml/g to 0.85 ml/g. The pore volume of α-Al₂O₃ can be measured, for example, by the BET method using nitrogen physisorption. If the pore volume of α-Al₂O₃ is less than 0.10 ml/g, it may not be easy to support the metal, and if it exceeds 0.85 ml/g, the activity of the supported metal may be reduced due to easy adsorption of moisture in the air.

The α-Al₂O₃ may have a lateral crushing strength of 20 N/particle to 200 N/particle. If the lateral crushing strength of α-Al₂O₃ is less than 20 N/particle, catalyst durability may be weakened during the process, and if it exceeds 200 N/particle, molding may not be easy in catalyst production. The lateral crushing strength of α-Al₂O₃ can be measured using, for example, the DLII Intelligent Particle Strength Tester manufactured by Dalian Chemical Industrial Research and Design Institute. Thirty carrier particles are randomly selected as samples, and their lateral crushing strength can be measured. The lateral crushing strength of the carrier can be obtained by calculating the average value.

The α-Al₂O₃ may have a primary particle diameter of 0.01 µm to 100 µm, for example, 0.1 µm to 20 µm, 0.5 µm to 10 µm, or 1 µm to 5 µm. The α-Al₂O₃ may have a secondary particle diameter of 0.1 µm to 1000 µm, for example, 1 µm to 500 µm, 10 µm to 200 µm, or 30 µm to 100 µm.

The carrier may include α-Al₂O₃, and crystalline silica alumina, amorphous silica, amorphous silica alumina, or a combination thereof. In this case, the carrier may contain α-Al₂O₃ as a main component. For example, the carrier may include 65 wt% to 99.9 wt% of α-Al₂O₃, and 0.1 wt% to 35 wt% of crystalline silica alumina, amorphous silica, amorphous silica alumina, or a combination thereof, based on a total weight of the carrier.

The carrier may further include crystalline silica alumina. An example of a method of including crystalline silica alumina in a carrier is a method of adding aluminosilicate as a raw material and calcining during production of the carrier. Additionally, in another way, pre-calcinated aluminosilicate mineral (crystalline silica alumina) can be added to the carrier raw material.

A content of the crystalline silica alumina in the carrier may be 0.01 wt% to 15 wt%, for example, for example 0.1 wt% to 13 wt%, or 5.0 wt% to 10 wt%, based on a total weight of the carrier.

Additionally, the carrier may further include at least one of amorphous silica or amorphous silica alumina. An example of a method of including amorphous silica or amorphous silica alumina in the carrier is a method of adding these components as raw materials and calcining during production of the carrier.

A content of amorphous silica in the carrier may be 0.01 wt% to 10 wt%, for example 0.1 wt% to 5 wt%, or 0.2 wt% to 3 wt%, based on a total weight of the carrier. Additionally, a content of amorphous silica alumina in the carrier may be 0.01 wt% to 10 wt%, for example 0.1 wt% to 5 wt%, or 0.2 wt% to 3 wt%, based on a total weight of the carrier.

The active metal is a component that has an activity that contributes to improving product yield when producing 1,3-cyclopentanediol by hydrogenating 4-hydroxy-2-cyclopentenone and is supported on a carrier.

The active metal may include Pt, Pd, Ru, Ir, Ni, Co, Cu, or a combination thereof, and may especially include Ru. When Ru is included as the active metal, the formation of 3-hydroxycyclopentanone, an intermediate, can be further reduced and the yield of 1,3-cyclopentanediol can be further increased.

As an example, Ru may be ruthenium from which the surface oxide layer has been removed. Ruthenium with the surface oxide layer removed refers to pure ruthenium with the oxide layer removed from the surface.

Generally, a native oxide layer surrounds ruthenium, which can inhibit the reaction. If the ruthenium catalyst is heat treated in the presence of hydrogen gas, the surrounding oxide layer can be removed. The heat treatment may be performed at a temperature of 200 °C or higher for 2 hours or more, but is not limited thereto.

When a catalyst containing ruthenium from which the surface oxide layer has been removed through heat treatment is added, hydrogen gas (H₂, H-H group) and an alkene hydrocarbon with a double bond are formed on the ruthenium surface during the formation of 1,3-cyclopentanediol. Hydrogen formed on the surface of ruthenium reacts with alkene hydrocarbons, and the alkene hydrocarbons become saturated hydrocarbons.

The process of generating saturated hydrocarbons on the surface of the ruthenium catalyst from which the surface oxide layer has been removed and the process of preparing 1,3-cyclopentanediol from 4-hydroxy-2-cyclopentenone occur simultaneously. Accordingly, 1,3-cyclopentanediol can be prepared in one step without forming the intermediate 3-hydroxycyclopentanone.

If ruthenium without the surface oxide layer is removed in the preparation of 1,3-cyclopentanediol, intermediates are formed due to reaction inhibition of the oxide layer around the catalyst, resulting in a yield of less than 40%. However, when a ruthenium catalyst from which the surface oxidation layer is removed is used, intermediates are not formed, and thus a yield of 1,3-cyclopentanediol can be increased to 45% or more.

A carbon number of saturated hydrocarbons and alkene hydrocarbons may be 2 to 10, preferably 2 to 5, but is not limited thereto.

The catalyst may include 0.1 wt% to 10 wt%, for example 0.5 wt% to 5 wt%, or greater than 0.5 wt% and 5 wt% or less of the active metal based on a total weight of the catalyst. If the content of the active metal is less than 0.1 wt%, reaction activity may be very low, and if it exceeds 10 wt%, the dispersion of the active metal may be significantly lowered, resulting in poor reaction activity.

A method for preparing 1,3-cyclopentanediol according to another embodiment includes preparing 1,3-cyclopentanediol by hydrogenating 4-hydroxy-2-cyclopentenone in the presence of a catalyst.

At this time, the catalyst used for a method for preparing 1,3-cyclopentanediol including a carrier including α-Al₂O₃ and an active metal supported on the carrier is the same as described above, and thus repeated descriptions are omitted.

As an example, the preparing of 1,3-cyclopentanediol may be accomplished by adding a catalyst to a solution of 4-hydroxy-2-cyclopentenone in an organic solvent.

The 4-hydroxy-2-cyclopentenone may be included in an amount of 0.01 wt% to 50 wt% based on a total weight of the solution. If the content of 4-hydroxy-2-cyclopentenone is less than 0.01 wt%, ring opening or side reactions may occur due to overreaction, and if it exceeds 50 wt%, decreased activity and self-polymerization of 4-hydroxy-2-cyclopentenone may occur during the reaction.

The organic solvent may include 2-methyltetrahydrofuran, methanol, acetone, or a combination thereof, for example, 2-methyltetrahydrofuran.

The catalyst may be added in an amount of 0.05 to 0.3 parts by weight, for example, 0.1 to 0.2 parts by weight, based on 100 parts by weight of 4-hydroxy-2-cyclopentenone. If the catalyst is added in less than 0.05 parts by weight, the double bond of the alkene hydrocarbon may not react and saturated hydrocarbons may not be formed. If it is added in excess of 0.3 parts by weight, because there are many unreacted catalysts, economic losses may occur, and ring opening or side reactions of cyclopentane may occur due to overreaction.

Since 1,3-cyclopentanediol is prepared by the hydrogenation reaction of 4-hydroxy-2-cyclopentenone, the preparing of 1,3-cyclopentanediol can be performed in a hydrogen (H₂) atmosphere.

In addition, the preparing of 1,3-cyclopentanediol may be performed in a hydrogen atmosphere at a temperature of 70 °C to 200 °C and a pressure of 10 bar to 70 bar for 10 minutes to 2 hours, and for example, the reaction may be performed at a temperature of 100 °C to 150 °C and a pressure of 30 bar to 50 bar for 30 minutes to 1 hour.

If the reaction temperature is less than 70 °C, the reaction may not occur, and if it exceeds 200 °C, ring opening of cyclopentane or side reactions may occur. In addition, if the reaction pressure is less than 30 bar or the reaction time is less than 30 minutes, the reaction may not occur due to a lack of H-H groups on the active metal surface, and if it exceeds 70 bar, ring opening of cyclopentane may occur. Additionally, if the reaction time exceeds 2 hours, the reaction may not proceed any further, making it uneconomical.

In addition, the method for preparing 1,3-cyclopentanediol may further include removing moisture from 4-hydroxy-2-cyclopentenone before adding the catalyst to 4-hydroxy-2-cyclopentenone.

The removing of moisture may be achieved by adding MgSO₄ to 4-hydroxy-2-cyclopentenone.

By removing moisture, when polymerizing using 1,3-cyclopentanediol as a monomer, the polymerization rate of the polymer can be controlled.

The method for preparing 1,3-cyclopentanediol may cause side reactions, and in the side reactions, by-products including cyclopentanone, cyclopentanol, 3-cyclopentanediol, or a combination thereof may be formed. These side reaction products are substances formed separately from intermediates. As mentioned above, the catalyst for preparing 1,3-cyclopentanediol includes α-Al₂O₃ with few acid points on the surface as a carrier, and thus there are few by-products during the hydrogenation reaction and selectivity for 1,3-cyclopentanediol is improved.

Therefore, 1,3-cyclopentanediol prepared by the method for preparing 1,3-cyclopentanediol may include 1 wt% or less, for example, 0.5 wt% or less, or 0.1 wt% to 0.5 wt% of by-products including cyclopentanone, cyclopentenol, 1,3-cyclopentanediol, or a combination thereof. If the content of the by-product exceeds 1 wt% based on the total weight of 1,3-cyclopentanediol, there may be a problem that the polymer synthesis reaction is stopped due to the by-product when 1,3-cyclopentanediol is used as a polymer polymerization monomer, and purity can be increased through additional purification processes.

Meanwhile, 4-hydroxy-2-cyclopentenone may be prepared from an aqueous solution of furfuryl alcohol, and furfuryl alcohol is a biomass-derived compound.

The furfuryl alcohol is a derivative of furfural extracted from plant materials such as corn cobs, oats, bran, rice bran, and sawdust.

As an example, 4-hydroxy-2-cyclopentenone can be prepared by adding a rearrangement reaction catalyst to an aqueous solution of biomass-derived furfuryl alcohol.

The preparing 4-hydroxy-2-cyclopentenone from an aqueous solution of furfuryl alcohol has the advantage of being cost-effective and environmentally friendly as it uses water as a solvent.

The rearrangement reaction catalyst may include calcium oxide, lead oxide, aluminum oxide, iron oxide, calcium chloride, zinc acetate, paratoluene sulfonic acid, stannous chloride, stannous sulfate, stannous oxide, stannic oxide, stannous octylate, tetraphenyl tin, a tin powder, titanium tetrachloride, or a combination thereof, and for example calcium oxide.

Additionally, the rearrangement reaction catalyst may be included in an amount of 0.1 to 10 parts by weight, for example, 0.1 to 5 parts by weight, based on 100 parts by weight of furfuryl alcohol. If the rearrangement reaction catalyst is included in less than 0.1 parts by weight, the rearrangement reaction may not occur and 4-hydroxy-2-cyclopentenone may not be produced, and if it exceeds 10 parts by weight, there will be a lot of unreacted catalyst, making it uneconomical.

As an example, 4-hydroxy-2-cyclopentenone can be prepared by adding a rearrangement reaction catalyst to an aqueous solution of furfuryl alcohol and then reacting at a temperature of 100 °C to 300 °C for 10 minutes to 1 hour.

As an example, the process of preparing 1,3-cyclopentanediol from biomass-derived furfuryl alcohol can be represented in Reaction Scheme 3.

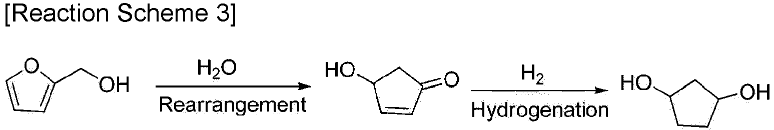

### DETAILED DESCRIPTION OF THE EMBODIMENTS

Hereinafter, specific examples of the invention are presented. However, the examples described below are only intended to specifically illustrate or describe the invention, and this should not limit the scope of the invention.

### [Preparation Example: Preparation of 1,3-Cyclopentanediol]

### (Example 1)

An aqueous solution was prepared by dissolving 14 g of furfuryl alcohol, a derivative of furfural extracted from biomass, in 685.97 mL of water. To the aqueous solution, 0.028 g of a calcium oxide catalyst was added and then, stirred at 200 °C for 0.5 hour to prepare 4-hydroxy-2-cyclopentenone.

A catalyst (Ru/α-Al₂O₃) having 5 wt% of a Ru content and supported on α-Al₂O₃ was prepared. Herein, the α-Al₂O₃ had an acid point of 0.001 mmol/g, a BET surface area of 7.2 m²/g, and a pore volume of 0.44 cm³/g. The α-Al₂O₃ used to prepare the catalyst was a product No. SA52238 manufactured by Saint-Gobain (France).

0.25 g of the catalyst was added to 4-hydroxy-2-cyclopentenone (a concentration: 10 wt%, a solvent: ethanol), and 50 bar of hydrogen was charged in a batch reactor. Herein, a reaction was performed at 120 °C for 1 hour. After completing the reaction, 1,3-cyclopentanediol was obtained.

### (Example 2)

An aqueous solution was prepared by dissolving 14 g of furfuryl alcohol, a derivative of furfural extracted from biomass, in 685.97 mL of water. To the aqueous solution, 0.028 g of a calcium oxide catalyst was added and then, stirred at 200 °C for 0.5 hour to prepare 4-hydroxy-2-cyclopentenone.

A catalyst (Ru/α-Al₂O₃) having 5 wt% of a Ru content and supported on α-Al₂O₃ was prepared. Herein, the α-Al₂O₃ had an acid point of 0.001 mmol/g, a BET surface area of 7.2 m²/g, and a pore volume of 0.44 cm³/g. The α-Al₂O₃ used to prepare the catalyst was a product No. SA52238 manufactured by Saint-Gobain (France).

0.25 g of the catalyst was added to 4-hydroxy-2-cyclopentenone (a concentration: 10 wt%, a solvent: ethanol), and 50 bar of hydrogen was charged in a batch reactor. Herein, a reaction was performed at 120 °C for 1 hour. After completing the reaction, 1,3-cyclopentanediol was obtained.

### (Example 3)

An aqueous solution was prepared by dissolving 14 g of furfuryl alcohol, a derivative of furfural extracted from biomass, in 685.97 mL of water. To the aqueous solution, 0.028 g of a calcium oxide catalyst was added and then, stirred at 200 °C for 0.5 hour to prepare 4-hydroxy-2-cyclopentenone.

A catalyst (Ru/α-Al₂O₃) having 5 wt% of a Ru content and supported on α-Al₂O₃ was prepared. Herein, the α-Al₂O₃ had an acid point of 0.001 mmol/g, a BET surface area of 7.2 m²/g, and a pore volume of 0.44 cm³/g. The α-Al₂O₃ used to prepare the catalyst was a product No. SA52238 manufactured by Saint-Gobain (France).

0.25 g of the catalyst was added to 4-hydroxy-2-cyclopentenone (a concentration: 10 wt%, a solvent: ethanol), and 50 bar of hydrogen was charged in a batch reactor. Herein, a reaction was performed at 120 °C for 1 hour. After completing the reaction, 1,3-cyclopentanediol was obtained.

### (Comparative Example 1)

An aqueous solution was prepared by dissolving 14 g of furfuryl alcohol, a derivative of furfural extracted from biomass, in 685.97 mL of water. To the aqueous solution, 0.028 g of a calcium oxide catalyst was added and then, stirred at 200 °C for 0.5 hour to prepare 4-hydroxy-2-cyclopentenone.

A catalyst (Ru/C) having 5 wt% of a Ru content and supported on activated carbon was prepared.

0.25 g of the catalyst was added to 4-hydroxy-2-cyclopentenone (a concentration: 10 wt%, a solvent: ethanol), and 50 bar of hydrogen was charged in a batch reactor. Herein, a reaction was performed at 120 °C for 1 hour. After completing the reaction, 1,3-cyclopentanediol was obtained.

### (Comparative Example 2)

An aqueous solution was prepared by dissolving 14 g of furfuryl alcohol, a derivative of furfural extracted from biomass, in 685.97 mL of water. To the aqueous solution, 0.028 g of a calcium oxide catalyst was added and then, stirred at 200 °C for 0.5 hour to prepare 4-hydroxy-2-cyclopentenone.

A catalyst (Ru/γ-Al₂O₃) having 5 wt% of a Ru content and supported on γ-Al₂O₃ was prepared. Herein, the γ-Al₂O₃ had an acid point of 0.8 mmol/g, a BET surface area of 187 m²/g, and a pore volume of 0.43 cm³/g. The γ-Al₂O₃ used to prepare the catalyst was a product manufactured by Sasol (Germany).

0.25 g of the catalyst was added to 4-hydroxy-2-cyclopentenone (a concentration: 10 wt%, a solvent: ethanol), and 50 bar of hydrogen was charged in a batch reactor. Herein, a reaction was performed at 120 °C for 1 hour. After completing the reaction, 1,3-cyclopentanediol was obtained.

### [Experimental Example: Conversion Rate and Yield of 1,3-Cyclopentanediol]

The 1,3-cyclopentanediols according to Examples 1 to 3 and Comparative Examples 1 to 2 were measured with respect to a conversion rate and a yield by using GC-FID (Agilent 7890B GC / flame ionization detector (FID)), and the results are shown in Table 1.

The reaction conditions for GC-FID are as follows.

### <Reaction condition of GC-FID>

Injector conditions: 250 °C, 4 µL, Split = 20 : 1
Column: HP-INNOWAX (30 m x 0.32 mm x 0.25 um)
Pressure rate: 2.33 mL/min
Mobile Phase: He, 1 mL/min
Oven temperature (Oven Temp.): From 80 °C, 10 °C/min to 320 °C, hold 5 min
Detector: Flame Ionized Detector
Injection method (Sampling): Direct injection

**(Table 1)**

| | Ru content (wt%) | Ru supported catalyst | Product yield (%) | | | |
|---|---|---|---|---|---|---|
| | | | HCPone^{a)} | CPone^{b)} | CPol^{c)} | CPdiol^{d)} |
| Comparative Example 1 | 5 | Ru/C | 0.0 | 0.0 | 3.4 | 96.5 |
| Comparative Example 2 | 5 | Ru/γ-Al₂O₃ | 0.0 | 0.0 | 7.2 | 92.8 |
| Example 1 | 5 | Ru/α-Al₂O₃ | 5.1 | 0.2 | 1.0 | 93.7 |
| Example 2 | 0.5 | Ru/α-Al₂O₃ | 27.8 | 0.0 | 2.3 | 69.9 |
| Example 3 | 1 | Ru/α-Al₂O₃ | 0.0 | 0.0 | 0.4 | 99.6 |

| | | | | | | |
|---|---|---|---|---|---|---|
| a) HCPone: Hydroxycyclopentanone b) CPone: cyclopentanone c) CPol: cyclopentanol d) CPdiol: 1,3-cyclopentanediol | | | | | | |

Referring to Table 1, when a catalyst supported on α-Al₂O₃ was applied to a hydrogenation reaction, a final product of 1,3-cyclopentanediol (CPdiol) was produced on the catalysts supported by 0.5 wt% to 5 wt% of a Ru metal according to Examples 1 to 3. In Examples 1 to 3, the reactant of 4-hydroxy-2-cyclopentenone (HCPenone) was 100% converted.

Comparing Example 3 with Comparative Example 1, even though Ru was included in a small content of 1%, 1,3-cyclopentanediol (CPdiol) was obtained at a yield of 99.6%. Accordingly, the Ru/α-Al₂O₃ catalyst may be advantageously used in the process by replacing a Ru catalyst supported on a carbon carrier.

While this invention has been described in connection with what is presently considered to be practical example embodiments, it is to be understood that the invention is not limited to the disclosed embodiments. On the contrary, it is intended to cover various modifications and equivalent arrangements included within the spirit and scope of the appended claims.

### [Industrial Applicability]

The present invention relates to a catalyst for preparing 1,3-cyclopentanediol, which is used to hydrogenate 4-hydroxy-2-cyclopentenone to prepare 1,3-cyclopentanediol.

## Claims

1. A catalyst for preparing 1,3-cyclopentanediol by hydrogenation of 4-hydroxy-2-cyclopentenone, comprising
a carrier including α-Al₂O₃, and
an active metal supported on the carrier.

2. The catalyst of claim 1, wherein
the α-Al₂O₃ has an acid point of 10 mmol/g or less when measured using an ammonia desorption method.

3. The catalyst of claim 1, wherein
the α-Al₂O₃ has
a specific surface area (BET) of 0.1 m²/g to 50.0 m²/g,
a pore volume of 0.10 ml/g to 0.85 ml/g, and
a lateral crushing strength of 20 N/particle to 200 N/particle.

4. The catalyst of claim 1, wherein
the carrier is the α-Al₂O₃, and
the carrier includes crystalline silica alumina, amorphous silica, amorphous silica alumina, or a combination thereof.

5. The catalyst of claim 4, wherein
the carrier includes, based on a total weight of the carrier,
65 wt% to 99.9 wt% of α-Al₂O₃, and
0.1 wt% to 35 wt% of crystalline silica alumina, amorphous silica, amorphous silica alumina, or a combination thereof.

6. The catalyst of claim 1, wherein
the active metal includes Pt, Pd, Ru, Ir, Ni, Co, Cu, or a combination thereof.

7. The catalyst of claim 1, wherein
the catalyst includes 0.1 wt% to 10 wt% of the active metal based on a total weight of the catalyst.

8. A method for preparing 1,3-cyclopentanediol, comprising
preparing 1,3-cyclopentanediol by hydrogenating 4-hydroxy-2-cyclopentenone in the presence of a catalyst,
wherein the catalyst includes
a carrier including α-Al₂O₃, and
an active metal supported on a carrier.

9. The method of claim 8, wherein
the preparing of the 1,3-cyclopentanediol is performed by adding a catalyst to a solution of 4-hydroxy-2-cyclopentenone in an organic solvent.

10. The method of claim 9, wherein
the 4-hydroxy-2-cyclopentenone is included in an amount of 0.01 wt% to 50 wt% based on a total weight of the solution.

11. The method of claim 9, wherein
the organic solvent includes 2-methyltetrahydrofuran, methanol, acetone, or a combination thereof.

12. The method of claim 8, wherein
in the preparing of 1,3-cyclopentanediol, 0.05 to 0.3 parts by weight of catalyst is added based on 100 parts by weight of 4-hydroxy-2-cyclopentenone.

13. The method of claim 8, wherein
the preparing of 1,3-cyclopentanediol is performed under a hydrogen atmosphere, a temperature of 70 °C to 200 °C, and a pressure of 10 bar to 70 bar.

14. The method of claim 8, wherein
the preparing of 1,3-cyclopentanediol is performed for 10 minutes to 2 hours.

15. The method of claim 8, wherein
the method for preparing 1,3-cyclopentanediol further includes preparing 4-hydroxy-2-cyclopentenone by adding a catalyst to an aqueous solution of furfuryl alcohol before the preparing of 1,3-cyclopentanediol.

16. The method of claim 15, wherein
the furfuryl alcohol is a biomass derived compound.

17. The method of claim 15, wherein
the catalyst includes calcium oxide, lead oxide, aluminum oxide, iron oxide, calcium chloride, zinc acetate, paratoluene sulfonic acid, stannous chloride, stannous sulfate, stannous oxide, stannic oxide, stannous octylate, tetraphenyl tin, a tin powder, titanium tetrachloride, or a combination thereof.

18. The method of claim 15, wherein
in the preparing of 4-hydroxy-2-cyclopentenone, 0.1 to 10 parts by weight of catalyst is added based on 100 parts by weight of furfuryl alcohol.

19. 1,3-Cyclopentanediol prepared by the method of any one of claim 8 to claim 18.

20. The 1,3-cyclopentanediol of claim 19, wherein
the 1,3-cyclopentanediol includes 1 wt% or less of by-products including cyclopentanone, cyclopentanol, 1,3-cyclopentanediol, or a combination thereof.
